# EUROPEAN PATENT APPLICATION

(11) **EP 4 546 352 A1**
(43) Date of publication of application: **30.04.2025**
(21) Application number: 24208122.2
(22) Date of filing: 22.10.2024
(51) Int. Cl.: G16B 20/30, G16B 40/00, G16B 40/10, G16B 40/20

(54) **PROTEIN INTERACTION PREDICTION DEVICE FOR UTILIZING UNOBSERVED PROTEIN COMPLEX TO GENERATE ANTIGEN PRESENTATION MODEL AND METHOD USING THE SAME**

(30) Priority: 23.10.2023 KR 20230142143
(71) Applicant: LG Management Development Institute, Seoul 07336 (KR)
(72) Inventor: Yim, Soorin, 07801 Seoul (KR); Hwang, Doyeong, 07524 Seoul (KR); Kim, Kiyoung, 07811 Seoul (KR)
(74) Representative: BCKIP Part mbB

(57) **Abstract**

A protein interaction prediction device utilizing unobserved protein complexes to generate an antigen presentation model includes a memory for storing protein complex-related datasets; and a processor operably connected to the memory, wherein the processor may be configured to: generate an embedding for at least one peptide by a pre-determined model based on at least one peptide generated through antigen processing, generate an embedding for at least one MHC, which is formed by being bound to the at least one peptide, identify results of mass spectrometry performed based on the at least one peptide and the at least one MHC, and train an antigen presentation model configured to predict the binding of the at least one peptide and the at least one MHC based on the results of the mass spectrometry.

## Description

### BACKGROUND

The present disclosure generally relates to a protein interaction prediction device for utilizing unobserved protein complexes to generate an antigen presentation model and a method using the same. More specifically, some embodiments of the present disclosure provide prediction results for unobserved antigen processing, peptide binding, and eluted ligand formation based on multiple mass spectrometry results performed in the same environment.

New types of antigens composed of protein complexes may be derived from somatic mutations and the like. In cancer cells, a protein that is not present in normal cells may be generated due to mutation or the like, and when the protein binds to major histocompatibility complex (MHC) to form a protein complex and acts as an antigen, it is referred to as a new antigen. Immunogenicity means that T cells are activated by recognizing a particular antigen. In order for T cells to develop immunogenicity, the T cell receptor and protein complex need to be able to be physically bound to each other.

Recently, the advancement of artificial intelligence has enabled the combination of various peptides that can form protein complexes. Given the peptide and MHC amino acid sequences, it may be possible to predict whether antigen processing will occur, leading to the formation of an eluted ligand.

Therefore, it is necessary to generate an antigen presentation model by constructing a more precise dataset based on various peptides and MHC amino acid sequences.

(Patent Document) Korean Patent No. 10-2389175

### SUMMARY

Some embodiments of the present disclosure may extract the structure of protein complexes derived from random peptides and MHC amino acid sequences, and repeatedly learn to generate a model that may predict eluted ligands.

According to certain embodiments of the present disclosure, a prediction device may classify a dataset used for training to generate a model capable of predicting eluted ligands.

The problems solved by the present disclosure are not limited to those mentioned above, and other problems not mentioned may be clearly understood by those skilled in the art from the description below.

A protein interaction prediction device utilizing unobserved protein complexes to generate an antigen presentation model according to an embodiment of the present disclosure includes a memory for storing protein complex-related datasets; and a processor electrically connected to the memory, wherein the processor may be configured to: generate an embedding for at least one peptide by a pre-determined model based on at least one peptide generated through antigen processing, generate an embedding for at least one MHC, which is formed by the binding of the at least one peptide, identify the results of mass spectrometry performed based on the at least one peptide and the at least one MHC, and train an antigen presentation model that predicts the binding of the at least one peptide and the at least one MHC based on the results of mass spectrometry.

In addition, a protein interaction prediction method utilizing unobserved protein complexes to generate an antigen presentation model, performed by a processor of a computer device according to the present disclosure, may include generating an embedding for at least one peptide by a pre-determined model based on at least one peptide generated through antigen processing; generating an embedding for at least one MHC, which is formed by binding of the at least one peptide; identifying the results of mass spectrometry performed based on the at least one peptide and the at least one MHC; and training an antigen presentation model that predicts the binding of the at least one peptide and the at least one MHC based on the results of mass spectrometry.

In addition, other method and system for implementing the present disclosure and a computer-readable recording medium storing a computer program for executing the method, may further be provided.

Furthermore, a computer program stored on a medium that allows the method of implementing certain embodiments of the present disclosure to be performed on a computer may further be provided.

According to an embodiment of the present application, a protein interaction prediction device may perform one or more processes to predict antigen processing, peptide-MHC binding, and eluted ligand formation through peptide and MHC amino acid sequences.

In addition, according to various embodiments of the present disclosure, a protein interaction prediction method can classify datasets used in each process into positive or negative and repeat learning to generate an antigen presentation model in order to achieve predictions that are difficult to implement or solve experimentally.

Effects of the present disclosure are not limited to those mentioned above, and another effects not mentioned may be clearly understood by those skilled in the art from the description below.

### BRIEF DESCRIPTION OF DRAWINGS

FIG. 1 is a schematic diagram of illustrating an overall process for generating an antigen presentation model utilizing a non-observed protein complex according to various embodiments of the present disclosure.
FIG. 2 is a table for showing input values of individual processes for generating an antigen presentation model utilizing a non-observed protein complex according to various embodiments of the present disclosure.
FIG. 3 is a table of a classification of a non-observed protein complex in a method of generating an antigen presentation model utilizing a non-observable protein complex according to various embodiments of the present disclosure.
FIG. 4 is a schematic block flow diagram of an antigen processing process in a method of generating an antigen presentation model according to various embodiments of the present disclosure.
FIG. 5 is a schematic block flow diagram of a peptide-MHC binding prediction process in a method of generating an antigen presentation model according to various embodiments of the present disclosure.
FIG. 6 is an overall block flow diagram of a method of generating an antigen presentation model according to various embodiments of the present disclosure.
FIG. 7 is an overall flowchart of a method of generating an antigen presentation model according to various embodiments of the present disclosure.
FIG. 8 is a data classification flowchart relating to an antigen processing process according to various embodiments of the present disclosure.
FIG. 9 is a flow diagram of data classification relating to a peptide-MHC binding prediction process according to various embodiments of the present disclosure.
FIG. 10 is a schematic block diagram of a protein interaction prediction device that utilizes a non-observed protein complex to generate an antigen presentation model according to various embodiments of the present disclosure.

### DETAILED DESCIPTION

Throughout the present disclosure, the same reference numerals designate the same components. The present disclosure does not describe all elements of the embodiments, and general contents in the technical field of the present disclosure or duplicated content among the embodiments are omitted. The terms "unit, module, member, block" used in the specification may be implemented in software or hardware, and depending on the embodiments, multiple "units, modules, members, blocks" may be implemented as a single component, or a single "unit, module, member, block" may also include multiple components.

Throughout the specification, when a part is described as being "connected" to another part, it includes not only cases where they are directly connected but also cases where they are indirectly connected, which may be connected by a wireless communication network.

Furthermore, when a part is described as "comprising" a certain component, it means that it may further include other components, not excluding other components unless explicitly stated otherwise.

Throughout the specification, when one member is described as being "on" other member, it includes not only cases where the members are in contact but also cases where another member exists between them.

Terms such as "first" and "second" are used to distinguish one component from another, and are not intended to limit the components to those aforementioned by the terms.

Singular expressions include plural expressions unless the context clearly indicates otherwise.

Identification numerals used for each step are provided for convenience in description and do not specify the order of the steps, and the each step may be carried out in a different order unless a specific order is explicitly described.

The operating principles and embodiments of the present disclosure will be described below with reference to the accompanying drawings.

The term "device according to the present disclosure" in this specification includes various devices capable of performing operations and providing results to users. For example, the device according to the present disclosure may include a computer, a server device, and a portable terminal, or it may take any one of these forms.

Here, the computer may include, for example, a notebook, a desktop, a laptop, a tablet PC, or a slate PC, equipped with a web browser.

The server device is a server that processes information by communicating with an external device, and may include an application server, a computing server, a database server, a file server, a game server, a mail server, a proxy server, and a web server.

The portable terminal is for example, a wireless communication device ensuring portability and mobility, and may include all kinds of handheld-based wireless communication devices such as Personal Communication System (PCS), Global System for Mobile communications (GSM), Personal Digital Cellular (PDC), Personal Handyphone System (PHS), Personal Digital Assistant (PDA), International Mobile Telecommunication (IMT)-2000, Code Division Multiple Access (CDMA)-2000, W-Code Division Multiple Access (W-CDMA), Wireless Broadband Internet (WiBro) terminal, smart phone, and wearable devices such as watches, rings, bracelets, anklets, necklaces, glasses, contact lenses, or head-mounted devices (HMD).

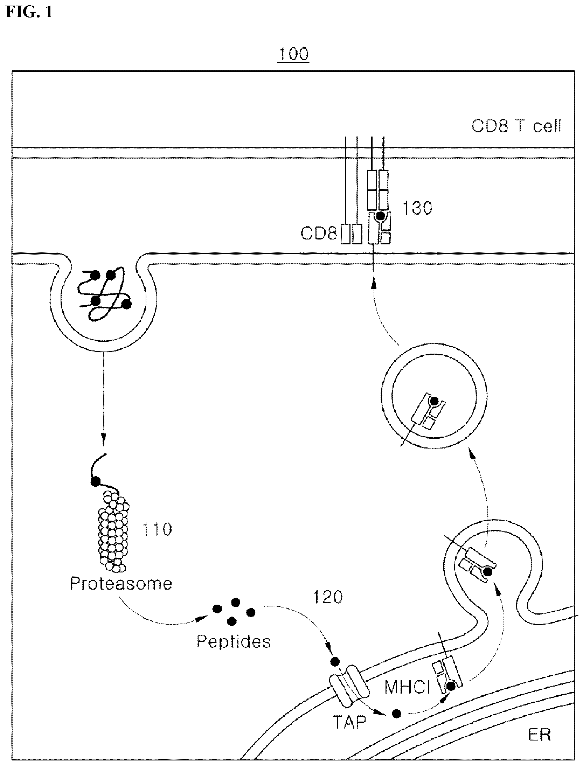

FIG. 1 is a schematic diagram of illustrating an overall process for generating an antigen presentation model utilizing a non-observed protein complex according to various embodiments of the present disclosure.

Referring to FIG. 1, a method for generating an antigen presentation model using a non-observed protein complex according to an embodiment may be performed through an overall process such as a process illustrated in an exemplary diagram 100. Antigen presentation may function primarily by antigen presenting cells (APCs), which are certain types of immune cells. Antigen presenting cells may include macrophages, B cells, and the like. Hospital bodies or other antigens that have entered into the human body from the outside of the body may be taken up by the antigen presenting cells and digested therein to degrade into small fragments (e.g., peptides).

According to an embodiment, these digested antigen fragments may bind to Major Histocompatibility Complex (MHC) molecules. The MHC molecules may be present on a cell surface and serve to stably present the antigen fragments. The formed MHC-antigen complex is exposed on the surface of the antigen presenting cells to allow immune cells to detect it. This mechanism may be referred to as MHC-Antigen Complex Presentation.

According to an embodiment, immune cells detect the MHC-antigen complex, identify the antigen, and in response, cytotoxic T cells may destroy the pathogen or the infected cells, or B cells may produce antibodies against the antigen to neutralize the pathogen. Through this antigen presentation process, the immune system can detect the invasion of pathogens and perform an appropriate defensive response.

According to an embodiment, immune processing 110 may refer to a process in which the immune system digests and degrades antigen molecules to recognize and respond to them. This process involves breaking down the antigen into small fragments so that immune cells can recognize and respond to them.

The immune processing 110 according to an embodiment may occur through two pathways. For instance, the immune processing 110 may occur through an endogenous pathway. The endogenous pathway is utilized to process antigens produced inside the cell. Protein antigens primarily produced inside the cell are degraded by intracellular proteolytic machinery. This degradant can be treated by a protein-degrading enzyme called a proteasome. Thereafter, an MHC-I antigen complex may be formed by binding to an MHC-I molecule. Th MHC-I antigen complex is exposed on the cell surface, allowing cytotoxic T cells to recognize it and perform a response. For another example, the immune processing 110 may occur through an exogenous pathway. The exogenous pathway is utilized to process external antigen that have been absorbed by the cell. The external antigen is absorbed into the cell and transported into an endosome. The endosome is used to digest and degrade the antigen, and the degraded fragments can bind to MHC- II molecules and form anMHC-II antigen complex. The MHC-II antigen complex is presented on the cell surface, allowing B cells and helper T cells to recognize and respond to it. The MHC-antigen complex formed through these pathways can be detected by immune cells to initiate an immune response. The immune processing 110 is one of processes of the immune system which allow various antigens to be effectively processed so that immune cells may recognize and respond to them.

Peptide-MHC binding 120 according to an embodiment may refer to a process in which a peptide, which corresponds to an antigen fragment in the immune system, is bound to major histocompatibility complex (MHC) molecules. This binding process may enable immune cells to detect the antigen fragment and carry out an appropriate immune response.

In the peptide-MHC binding 120 according to an embodiment, MHC molecules are present on the cell surface to stably indicate antigen fragments, allowing immune cells to recognize them. The MHC molecules may be categorized into MHC class I(MHC-I) molecules and MHC class II(MHC- II) molecules. For example, MHC-I molecules are present in almost all nucleated cells and present antigen fragments produced within the cell. These antigen fragments are degraded and generated inside the cell by proteasomes and other proteolytic enzymes. The antigen fragments are then bound to the MHC-I molecules in the endoplasmic reticulum (ER) to form an MHC-I antigen complex. On the other hand, the MHC-II molecules are primarily involved in processing antigens that are taken into the cell and degraded within an endosome. The degraded fragments are bound to the MHC-II molecules to form an MHC-II antigen complex. The peptide-MHC binding 120 is a process through which specific structures of antigen fragments interact with binding sites on MHC molecules to form a stable binding. This process allows antigen fragments to be effectively detected by immune cells.

According to an embodiment, an eluted ligand 130 may be a key concept in protein-protein interaction studies or antigen presentation processes. The eluted ligand 130 may refer to a state in which a molecule is released after being bound to a target protein or molecule. For example, in protein-protein interaction, a complex may be formed when one protein is bound to another protein. In this example, the eluted ligand 130 may refer to a protein or molecule that is dissociated from a complex formed when one protein is bound to another. This separation or elution may be an experimental procedure and may be refer to a process of releasing the binding under specific conditions. In the antigen presentation process, an antigen protein may be bound to major tissue-fit type molecules to form an MHC-antigen complex. This MHC-antigen complex is detected and recognized by immune cells, and when the antigen protein is released or dissociated from the MHC molecule, then it is referred to as the eluted ligand 130. The eluted ligand 130 may be predicted through the generation of the antigen presentation model as described in some embodiments of the present disclosure.

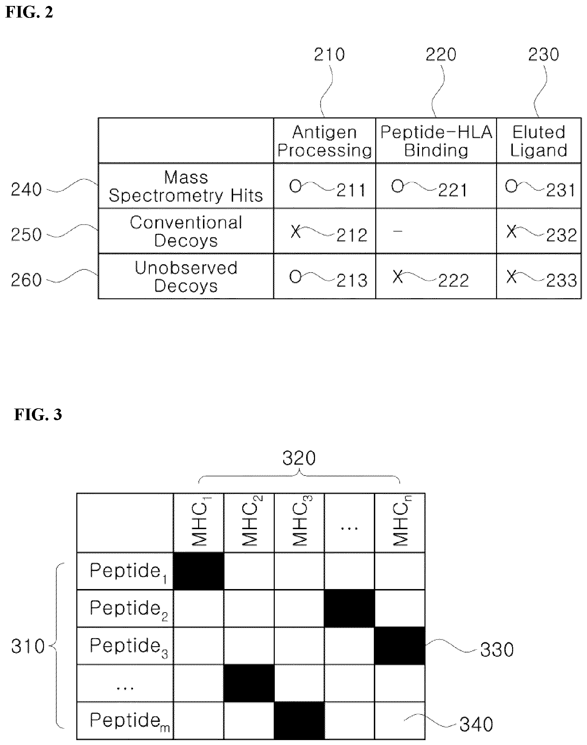

FIG. 2 is a table for showing input values for individual processes in generating an antigen presentation model utilizing unobserved protein complexes according to various embodiments of the present disclosure.

Referring to FIG. 2, each of a process 210 of antigen or immune processing, a process 220 of peptide-MHC binding, and a prediction process 230 of eluted ligand 230 may be performed for at least one peptide and at least one MHC amino acid sequence as input through a pre-established model.

The process 210 for immune processing according to an embodiment may perform a process by using a first result 211 and unobserved protein complex 213 as positive datasets, and by using protein complex corresponding to the structure of protein complexes including a peptide utilized for extracting the first result as negative datasets. The positive dataset in the process 210 of the immune processing 210 is the first result 211, which may be one of the outcomes of mass spectrometry hits 240, and the first result 211 may have been derived from the peptide. The first result 211 may be an outcome corresponding to the structure of the protein complex stored in memory among the results of mass spectrometry, and a processor may confirm the unobserved protein complex 213 among the protein complex-related datasets stored in the memory. The positive dataset in the process 210 of the immune processing may utilize unobserved protein complexes as unobserved decoys 260. The unobserved protein complex 213 may be a complex that was not bound to MHC. The negative dataset in the process 210 of the immune processing may utilize conventional protein complexes as conventional decoys 250. The negative dataset in the process 210 of the immune processing may be a protein complex corresponding to a protein complex predicted from peptide embeddings among the protein complex-related datasets. In other words, the structure of protein complexes derived from specific peptides may already be known. The process 210 of immune processing may learn these already known protein complexes 212 as negative datasets.

The process 220 of peptide-MHC binding according to an embodiment may perform a process using a second result 221 as a positive dataset and a third result 222 as a negative dataset. In the process 220 of peptide-MHC binding, the positive dataset is the second result 221, which may be one of the mass spectrometry hits 240. The second result 221 may be obtained from a peptide and correspond to the structure of a protein complex among the peptide-MHC complexes in which at least one peptide and at least one MHC are bound. In the process 220 of peptide-MHC binding, the negative dataset is the third result 222, which may not correspond to the structure of a protein complex among the mass spectrometry results. For example, the third result 222 may correspond to a dataset that was not bound to MHC among the positive datasets of the process 210 of the immune processing. That is, the third result 222 may not be bound to MHC in unobserved decoy 260.

The prediction process 230 of the eluted ligand according to an embodiment may perform a process using the first result 211 and/or the second result 221 as a positive dataset, and using a protein complex 232 corresponding to the structure of a protein complex predicted from the peptide embeddings, and the third result 233 as a negative dataset. In the prediction process 230 of the eluted ligand, the positive dataset may be the first result 211 and/or the second result 221. In other words, the positive dataset in the prediction process 230 of the eluted ligand may correspond to both the positive dataset in the process of the immune processing 210 and the positive dataset in the process 220 of the peptide-MHC binding. In the prediction process 230 of the eluted ligand, the negative dataset may correspond to both the negative dataset in the immune processing 210 process and a dataset in the positive dataset of the process 210 of the immune processing that was not bound to MHC.

FIG. 3 is a table of a classification of a non-observed protein complex in a method of generating an antigen presentation model utilizing a non-observable protein complex according to the present disclosure.

Referring to FIG. 3, at least one peptide 310 in an embodiment of the present disclosure may comprise a first peptide 1 (Peptide₁), a second peptide 2 (Peptide₂), and up to a m-th peptide m (Peptideₘ). At least one MHC (320) in an embodiment of the present disclosure may comprise a first MHC (MHCi), a second MHC (MHC₂), up to a n-th MHC (MHCₙ).

According to an embodiment, the processor of the present disclosure may read data in the form of a table, such as a table in FIG. 3, from the memory during the process 210 of the immune processing, the binding process 220 of the peptide-MHC, and the prediction process 230 of the eluted ligand. For example, the data in table form in FIG. 3 may include mass spectrometry hits 330 and data 340 about unobserved protein complexes (unobserved decoy). Such data may be stored in the memory as part of the protein complex-related datasets.

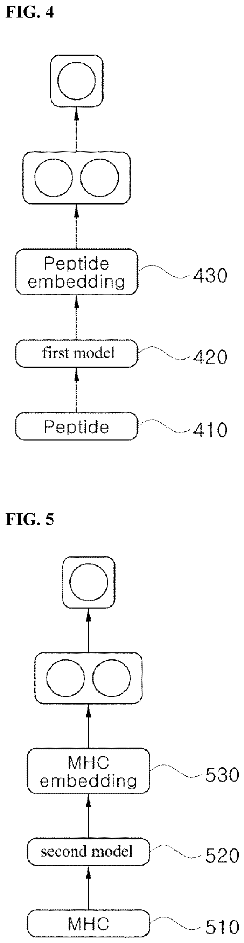

FIG. 4 is a schematic block flow diagram of an antigen processing process in a method of generating an antigen presentation model according to various embodiments of the present disclosure.

Referring to FIG. 4, the processor may generate an embedding 430 for at least one peptide through a first model 420 (e.g., a predetermined model) based on at least one peptide 410. The first model 420 may, for example, but not limited to, a Bidirectional Gated Recurrent Unit (Bi-GRU) 4. For instance, the first model 420 may be a model having four layers of a Bidirectional Gated Recurrent Unit. Each GRU layer of the first model 420 may update and pass information to a next layer while sequentially processing sequences. The first model 420 may be a deep learning model mainly used for sequence data processing, which may perform learning using at least one peptide 410 as an input value. The sequence of at least one peptide 410 can be an input value.

According to an embodiment, the processor may receive the sequence data of at least one peptide 410 as input for learning. At least one peptide 410 may comprise a continuous sequence of amino acids, and the processor may generally map these amino acids to a unique integer or embedding vector to represent a sequence numerically.

According to an embodiment, the processor may add an embedding layer that converts an amino acid of at least one peptide 410 sequence into an embedding vector. This embedding vector may be utilized as an input of the first model 420 by converting each of the amino acids into a high-dimensional vector.

According to an embodiment, the processor may select or grasp and predict the characteristics of at least one peptide 410 sequence by using the output of the GRU layer for each point in time in an output layer of the first model 420. At this case, the processor may be configured to vary the form of the output layer and the quantization function according to the classification, structure prediction, or the like of at least one peptide 410.

The processor according to an embodiment may train the first model 420 by using the prepared peptide data and correct answer data. The processor may give at least one peptide sequence including at least one peptide 410 as an input value in a learning process, and use correct answer data having a property or class of peptide as an output value. Then, when the learning is completed, the processor may give a peptide sequence as an input, obtain the output of the first model 420, and confirm the predicted value of the peptide. Accordingly, the processor may analyze the performance and the prediction result of the first model 420 to find an improvement point and perform the improvement. In an embodiment of the present disclosure, the processor may generate a peptide embedding 430 for at least one peptide from at least one peptide 410 via the first model 420, and perform an immune treatment process based thereon.

FIG. 5 is a schematic block flow diagram of a peptide-MHC binding prediction process in a method of generating an antigen presentation model according to various embodiments of the present disclosure.

Referring to FIG. 5, the processor may generate an embedding 530 for MHC through a second model 520 (e.g., a predetermined model) based on an MHC 510. Here, the second model 520 may refer to a 1D Convolution model. The second model 520 may be one of deep learning architectures configured to process data such as MHC sequences. The second model 520 may be an extension of the concept of the 2D Convolution model used for image processing and applied to the first-dimensional data. The second model 520 may include an input layer, a 1D convolution layer, an activation function, a pooling layer, and a pulley connected layer. The input layer may be given an MHC sequence as an input, the 1D Convolution layer may extract a local pattern of input data, and the activation function may apply an activation function to the 1D Convolution layer to add nonlinearity, where the Rectified Linear Unit (ReLu) function may be used. The pooling layer may down-sample data through a pooling operation to summarize important information, and the pulley connected layer is a density layer, and may perform final prediction by utilizing extracted features. That is, the second model 520 may be utilized in a peptide-MHC binding process by effectively sensing and extracting regional patterns of sequence data of the MHC 510.

The processor according to an embodiment may receive the sequence data of the MHC 510 as an input value for learning. The MHC 510 may comprise a contiguous sequence of amino acids, and the processor may generally map the amino acids to a unique integer or embedding vector to numerically represent a sequence.

According to an embodiment, the processor may add an embedding layer that converts an amino acid of the sequence of the MHC 510 into an embedding vector. This embedding vector may be utilized as an input of the second model 520 by converting each of the amino acids in the sequence of the MHC 510 into a high-dimensional vector. As a result, the processor may generate an embedding 530 for the MHC via the second model 520. The processor may generate an embedding for at least one peptide via the first model based on at least one peptide, and utilize the MHC embedding 530 to predict whether peptide-MHC binding occurs.

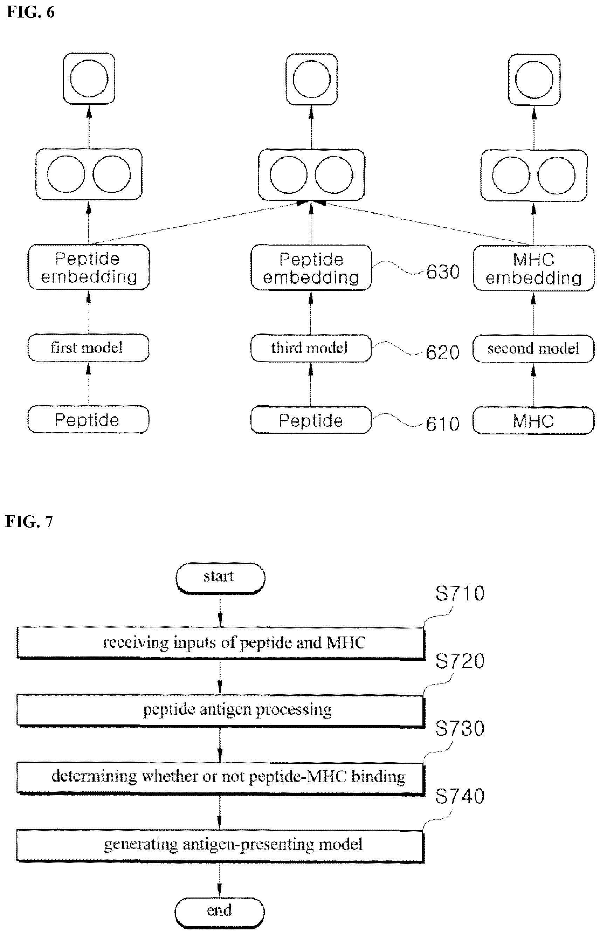

FIG. 6 is an overall block flow diagram of a method of generating an antigen presentation model according to various embodiments of the present disclosure.

Referring to FIG. 6, the processor may generate an embedding 630 for at least one peptide based on at least one peptide 610 via a third model 620. The third model 620 may be implemented with a Bi-GRU 4 that is the same as or similar to the first model.

The processor according to an embodiment can predict whether an eluted ligand is present by utilizing two types of peptide embeddings and one type of MHC embedding. Such predictions may be made through the antigen presentation models of some embodiments of the present disclosure. The two types of peptide embeddings may include a first peptide embedding (e.g., the embedding 430 for at least one peptide of FIG. 4) and an embedding 630 for at least one peptide. That is, the processor generates an antigen presentation model that predicts an eluted ligand, as shown in FIG. 6 together with FIGS. 4 and 5.

FIG. 7 is an overall flowchart of a method of generating an antigen presentation model according to various embodiments of the present disclosure.

In step S710, the processor may receive at least one peptide and at least one MHC amino acid sequence as input. The processor may read a protein complex related dataset from the memory and may receive a random peptide and a random MHC amino acid sequence as input values to generate an antigen presentation model.

In step S720, the processor may perform an antigen processing process on at least one peptide. The antigen processing process may be a process of digesting and degrading antigen molecules to recognize and react to antigens in an immune system. The present disclosure mainly describes an enclosed route. Peptides in the present disclosure can be understood as antigen molecules or antigen fragments.

In step S730, the processor may perform a peptide-MHC binding process. The peptide-MHC binding process may be a process in which an antigen fragment (e.g., peptide) is bound to a major histocompatibility type molecule in an immune system. When the antigen presenting model is generated, the peptide-MHC binding process may perform a process of classifying a dataset that is to perform learning according to whether the peptide is MHC bound or not into positive or negative.

In step S740, the processor may generate an antigen presentation model. The antigen presenting model may be a model that predicts an eluted ligand. The eluted ligand may refer to a state in which a molecule is bound to a specific protein or molecule during protein interaction, and then the binding is released. The processor according to some embodiments of the present disclosure may generate an antigen presentation model with a model that predicts an eluted ligand, and may classify in detail a set of data, which are to be subjected to an immune treatment process and a peptide-MHC binding process to generate the antigen presentation model, into positive or negative.

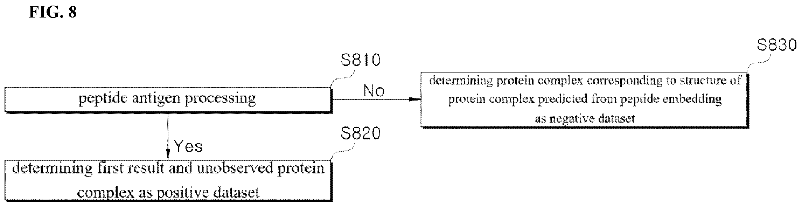

FIG. 8 is a data classification flowchart of an antigen processing process according to various embodiments of the present disclosure.

In step S810, the processor may perform an antigen processing process on at least one peptide. Step S810 is operated or performed in the same or similar way as or to step S720 of FIG. 7.

In step S820, the processor may determine a set of positive data that is utilized to perform the antigen processing process. In step S810, the processor may determine the first resultant and the non-observed protein complex as a positive dataset through antigen processing on at least one peptide. As a result, the processor can perform cumulative learning on the positive dataset to confirm the result value for the input as a correct answer.

In step S830, the processor may determine a negative dataset to be utilized to perform the antigen processing process. In step S810, the processor may determine the protein complex corresponding to the structure of the protein complex predicted from the embedding for at least one peptide as a negative dataset. As a result, the processor may perform cumulative learning on the negative dataset to identify a result value of the input as an incorrect answer.

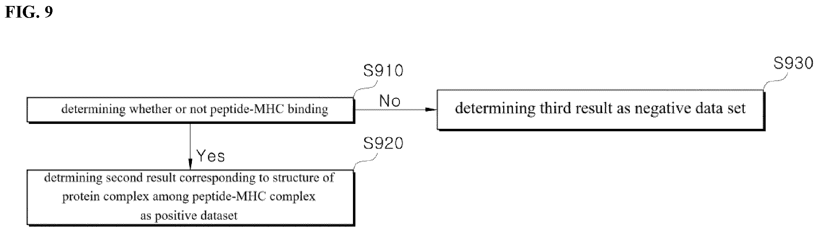

FIG. 9 is a flowchart for data classification of a peptide-MHC binding prediction process according to various embodiments of the present disclosure.

In step S910, the processor may perform a peptide-MHC binding process. Step S810 is operated or performed in the same or similar way as or to step S730 of FIG. 7.

In step S920, the processor may determine a set of positive data utilized to perform the peptide-MHC binding process. In step S910, the processor may determine a second result corresponding to the structure of a protein complex in the peptide-MHC complex bound to at least one peptide and the MHC as the positive dataset. As a result, the processor can perform cumulative learning on the positive dataset to confirm a result value for the input as a correct answer.

In step S930, the processor may determine a negative dataset utilized to perform the peptide-MHC binding process. In S910, the processor may determine a third result to which at least one peptide and the MHC are not bound as a negative set of data. As a result, the processor may perform cumulative learning on the negative dataset to identify a result value for the input in an incorrect answer.

The processor according to an embodiment may perform cross-learning using multiple embeddings for at least one peptide and MHC embeddings through the processes of verifying correct and incorrect responses and refining the positive and negative datasets as shown in FIGS. 8 and 9. In this case, the dataset used by the processor may be refined as positive or negative by crossing the datasets learned through the antigen processing process and the peptide-MHC binding process. The processor may ultimately generate an antigen presentation model to determine if an eluted ligand may be formed, utilizing the dataset obtained through antigen processing and peptide-MHC binding processes.

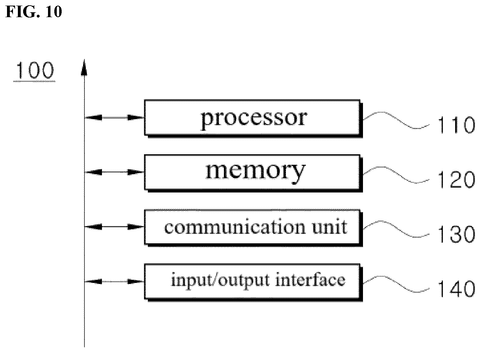

FIG. 10 is a block diagram of a protein interaction prediction device utilizing unobserved protein complexes to generate the antigen presentation model according to various embodiments of the present disclosure.

A device 1000 for predicting protein interaction may include a processor 1010, memory 1020, a communicator or communication unit 1030, and an input and/or output interface 1040, and the like. However, these internal components included in the device 1000 for predicting protein interaction are not limited thereto. Alternatively or additionally, the device 1000 for predicting protein interaction according to an embodiment of the present disclosure may perform the functions of the processor 1010 through a separate processing server or a cloud server instead of a processor.

Referring to FIG. 10, the processor 1010 may be configured to perform one or more operations of the device 1000 for predicting protein interaction in association with the memory 1020 that stores data on an algorithm for controlling the operation of components included in or associated with the device 1000 for predicting protein interaction or a program that reproduces the algorithm, and the data stored in the memory 1020. For example, the processor 1010 and memory 1020 may be implemented as separate chips. Alternatively, the processor 1010 and the memory 1020 may be implemented as an integrated single chip.

The processor 1010 may control one or more of the components described above to implement various embodiments according to the present disclosure in the device 1000 for predicting protein interaction, as described in embodiments illustrated in FIGS. 1 to 9.

The memory 1020 according to an embodiment may store data performing or supporting various functions of the device 1000 for predicting protein interaction, programs for the operation of the processor 1010, input/output data (e.g., images, videos, etc.), multiple application programs or applications running on the device for predicting protein interaction 1000, and data or instructions for operating the device 1000 for predicting protein interaction. At least some of these application programs may be downloaded from an external server via wired or wireless communication.

The memory 1020 may include at least one type of storage medium, such as flash memory type, hard disk type, Solid State Disk (SSD) type, Silicon Disk Drive (SDD) type, multimedia card micro type, card type memory (e.g., SD or XD memory), random access memory (RAM), static random access memory (SRAM), read-only memory (ROM), electrically erasable programmable read-only memory (EEPROM), programmable read-only memory (PROM), magnetic memory, magnetic disk, and optical disk. Additionally, the memory may be a database that is separate from the device for predicting protein interaction 1000, but is connected wired or wirelessly.

The communicator or communication unit 1030 according to an embodiment may include one or more components configured to communicate with external devices, including, for example, at least one of a broadcast receiving module or broadcast receiver, wired communication module or wired communicator, wireless communication module or wireless communicator, short-range communication module or short-range communicator, or location information module.

The wired communication module may include various wired communication modules such as a Local Area Network (LAN) module, a Wide Area Network (WAN) module, or a Value Added Network (VAN) module, as well as various cable communication modules such as Universal Serial Bus (USB), High Definition Multimedia Interface (HDMI), Digital Visual Interface (DVI), recommended standard 232 (RS-232), power line communication, or plain old telephone service (POTS).

The wireless communication module may include not only a WiFi module, a Wireless broadband (WiBro) module, but also a wireless communication module supporting various wireless communication methods such as Global System for Mobile Communication (GSM), Code Division Multiple Access (CDMA), Wideband Code Division Multiple Access (WCDMA), Universal Mobile Telecommunications System (UMTS), Time Division Multiple Access (TDMA), Long Term Evolution (LTE), 4G (Generation), 5G, and 6G.

The short-range communication module is for short-range communication and may support short-range communication using at least one of the following technologies: Bluetooth^{™}, Radio Frequency Identification (RFID), Infrared Data Association (IrDA), Ultra Wideband (UWB), ZigBee, Near Field Communication (NFC), Wireless-Fidelity (Wi-Fi), Wi-Fi Direct, or Wireless Universal Serial Bus (Wireless USB).

The input and/or output interface 1040 according to an embodiment serves as a channel for connecting various types of external devices to the device 1000 for predicting protein interaction. The input and/or output interface 1040 may include, for example, but not limited to, at least one of the following: a wired and/or wireless headset port, an external charger port, a wired/wireless data port, a memory card port, a port for connecting a device equipped with a Subscriber Identification Module (SIM), an audio Input/Output (I/O) port, a video Input/Output (I/O) port, or an earphone port. The device 1000 for predicting protein interaction may perform control related to the external device connected to the input and/or output interface 1040.

At least one component may be added or omitted in accordance with the performance of the components shown in FIG. 10. It will be readily understood by those skilled in the art that the mutual positions of the components may also be modified in accordance with the performance or structure of the device.

Meanwhile, each module or component shown in FIG. 10 represents software and/or hardware components such as a Field Programmable Gate Array (FPGA) and an Application Specific Integrated Circuit (ASIC).

Meanwhile, some embodiments of the present disclosure may be implemented in the form of a recording medium that stores instructions executable by a computer. The instructions may be stored in the form of program code and, when executed by a processor, may generate program modules to perform the operations of the disclosed embodiments. The recording medium may be implemented as a computer-readable recording medium.

The computer-readable recording medium includes any type of recording medium in which instructions that may be decrypted by a computer are stored. For example, Examples include a read only memory (ROM), a random access memory (RAM), a magnetic tape, a magnetic disk, a flash memory, an optical data storage device, and the like.

As described above, the disclosed embodiments are described with reference to the accompanying figures. A person of ordinary skill in the art may understand that the present disclosure may be implemented in a different form from the disclosed embodiments without changing the technical sprit or essential features of the present disclosure. The disclosed embodiments are illustrative and restrictive.

### [Explanation of Symbols]

1000: protein interaction prediction device
1010: processor
1020: memory
1030: communication unit
1040: input/output interface

## Claims

1. A system comprising:
memory for storing protein complex-related datasets; and
a processor operably connected to the memory, the processor configured to:
generate an embedding for at least one peptide by a pre-determined model based on at least one peptide generated through antigen processing,
generate an embedding for at least one MHC which is formed by being bound to the at least one peptide,
identify results of mass spectrometry performed based on the at least one peptide and the at least one MHC, and
train an antigen presentation model configured to predict binding of the at least one peptide and the at least one MHC based on the results of the mass spectrometry.

2. The system of claim 1, wherein:
the processor is configured to:
identify a first result corresponding to a structure of a protein complex from the results of the mass spectrometry,
identify an unobserved protein complex from the protein complex-related dataset stored in the memory, and
determine the first result and the unobserved protein complex as a positive dataset.

3. The system of claim 2, wherein:
the processor is configured to:
determine a protein complex, predicted from an embedding for a first peptide from the protein complex-related datasets stored in the memory, as a negative dataset, and
predict whether an antigen is processed based on the embedding for the at least one peptide.

4. The system of claim 3, wherein:
the processor is configured to:
identify a second result corresponding to the structure of the protein complex from the results of mass spectrometry, and
determine the second result as the positive dataset.

5. The system of claim 4, wherein:
the second result corresponds to the structure of the protein complex in a peptide-MHC complex formed by binding the at least one peptide and the at least one MHC to each other.

6. The system of claim 5, wherein:
the processor is configured to:
identify a third result containing the unobserved protein complex from the protein complex-related dataset stored in the memory, and
determine the third result as the negative dataset.

7. The system of claim 6, wherein:
the third result is associated with the at least one peptide and the at least one MHC being unbound to each other.

8. The system of claim 7, wherein:
the processor is configured to:
generate an embedding for the at least one MHC through another model based on the at least one MHC, and
predict whether the at least one peptide and the at least one MHC are bound to each other based on the embedding for the at least one peptide and the embedding for the at least one MHC.

9. The system of claim 8, wherein:
the processor is configured to:
determine, as the negative dataset, the third result and a protein complex corresponding to a structure of a protein complex predicted from the embedding for the at least one peptide from the protein complex-related dataset, and
predict whether an eluted ligand is formed based on the embedding for the at least one peptide and the embedding for the at least one MHC.

10. A computer-implemented method comprising:
generating an embedding for at least one peptide by a pre-determined model based on at least one peptide generated through antigen processing;
generating an embedding for at least one MHC which is formed by being bound to the at least one peptide;
identifying results of mass spectrometry performed based on the at least one peptide and the at least one MHC; and
training an antigen presentation model configured to predict binding of the at least one peptide and the at least one MHC based on the results of the mass spectrometry.

11. The computer-implemented method of claim 10, wherein:
the processor:
identifies a first result corresponding to a structure of a protein complex from the results of the mass spectrometry,
identifies an unobserved protein complex from protein complex-related datasets stored in memory, and
determines the first result and the unobserved protein complex as a positive dataset.

12. The computer-implemented method of claim 11, wherein:
the processor:
determines a protein complex predicted from an embedding for a first peptide from the protein complex-related datasets stored in the memory as a negative dataset, and
predicts whether an antigen is processed based on the embedding for the at least one peptide.

13. The computer-implemented method of claim 12, wherein:
the processor:
identifies a second result corresponding to the structure of the protein complex from the results of mass spectrometry, and
determines the second result as the positive dataset.

14. The computer-implemented method of claim 13, wherein:
the second result corresponds to the structure of the protein complex in a peptide-MHC complex formed by binding the at least one peptide and the at least one MHC to each other.

15. A non-transitory computer-readable storage medium having instructions that, when executed by one or more processors, cause the one or more processors to:
generate an embedding for at least one peptide by a pre-determined model based on at least one peptide generated through antigen processing,
generate an embedding for at least one MHC which is formed by being bound to the at least one peptide,
identify results of mass spectrometry performed based on the at least one peptide and the at least one MHC, and
train an antigen presentation model configured to predict binding of the at least one peptide and the at least one MHC based on the results of the mass spectrometry.
